(19) Europäisches Patentamt
European Patent Office
Office européen des brevets

(11) Veröffentlichungsnummer: **0 255 601 B1**

(12) **EUROPÄISCHE PATENTSCHRIFT**

(45) Veröffentlichungstag der Patentschrift: **11.09.91**

(51) Int. Cl.[5]: **C07D 413/04**, A01N 43/84

(21) Anmeldenummer: **87109174.0**

(22) Anmeldetag: **26.06.87**

(54) **Dialkylmaleinimide, Verfahren zu ihrer Herstellung und ihre Verwendung als Herbizide.**

(30) Priorität: **07.07.86 JP 157890/86**

(43) Veröffentlichungstag der Anmeldung:
**10.02.88 Patentblatt 88/06**

(45) Bekanntmachung des Hinweises auf die Patenterteilung:
**11.09.91 Patentblatt 91/37**

(84) Benannte Vertragsstaaten:
**BE CH DE FR GB IT LI NL**

(56) Entgegenhaltungen:
**EP-A- 0 170 191**

(73) Patentinhaber: **NIHON BAYER AGROCHEM K.K.**
**7-1, Nihonbashi Honcho 2-chome Chuo-ku**
**Tokyo 103(JP)**

(72) Erfinder: **Kume, Toyohiko**
**6-7-8, Asahigaoka**
**Hino-shi Tokyo(JP)**
Erfinder: **Goto, Toshio**
**3454-21, Honmachida**
**Machida-shi Tokyo(JP)**
Erfinder: **Kamochi, Atsumi**
**2-24-10, Higashi-Toyoda**
**Hino-shi Tokyo(JP)**
Erfinder: **Yamaguchi, Naoko**
**4-6-7, Shinmei**
**Hino-shi Tokyo(JP)**
Erfinder: **Yanagi, Akihiko**
**2-1200-1, Oume-shi**
**Tokyo(JP)**
Erfinder: **Hayakawa, Hidenori**
**1631-13, Shimokuzawa**
**Sagamihara-shi Kanagawa-ken(JP)**
Erfinder: **Yagi, Shigeki**
**1-30-7, Izumi, Suginami-ku**
**Tokyo(JP)**

(74) Vertreter: **Schumacher, Günter, Dr. et al**
**c/o Bayer AG Konzernverwaltung RP Patentabteilung**
**W-5090 Leverkusen 1 Bayerwerk(DE)**

**EP 0 255 601 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Dialkylmaleinimide, Verfahren zu ihrer Herstellung und ihre Verwendung als Herbizide.

Es wurde bereits offenbart, daß bestimmte Tetrahydrophthalimide herbizide Aktivitäten besitzen (siehe die JP-OS 30586/1986).

Nunmehr wurden neue Dialkylmaleinimide der Formel (I)

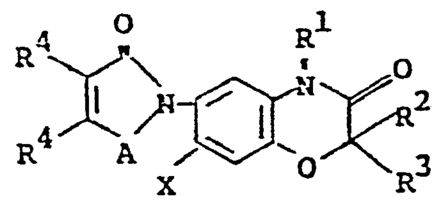

(I)

gefunden. In der Formel bezeichnen

A

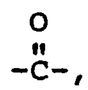, 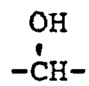 oder 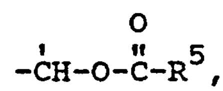,

worin $R^5$ eine Alkyl-Gruppe mit 1 bis 4 Kohlenstoff-Atomen oder eine Phenyl-Gruppe darstellt,

$R^1$ ein Wasserstoff-Atom, eine Alkyl-Gruppe mit 1 bis 4 Kohlenstoff-Atomen, eine Alkenyl-Gruppe mit 2 bis 4 Kohlenstoff-Atomen, eine Alkinyl-Gruppe mit 2 bis 4 Kohlenstoff-Atomen, eine Benzyl-Gruppe, eine Alkoxyalkyl-Gruppe mit insgesamt 2 bis 6 Kohlenstoff-Atomen, eine Cyanomethyl-Gruppe, eine Trimethylsilylmethyl-Gruppe, eine Alkylthioalkyl-Gruppe mit insgesamt 2 bis 6 Kohlenstoff-Atomen oder die Gruppe

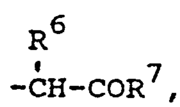,

worin $R^6$ ein Wasserstoff-Atom oder eine Alkyl-Gruppe mit 1 bis 2 Kohlenstoff-Atomen darstellt und $R^7$ eine Alkoxy-Gruppe mit 1 bis 4 Kohlenstoff-Atomen, eine Cycloalkoxy-Gruppe mit 3 bis 7 Kohlenstoff-Atomen, eine Halogenoalkoxy-Gruppe mit 1 bis 4 Kohlenstoff-Atomen, eine Alkylamino- oder Dialkylamino-Gruppe mit insgesamt 2 bis 6 Kohlenstoff-Atomen, eine N-($C_1$-$C_4$) Alkyl-N-phenylamino-Gruppe oder eine Trimethylsilylmethoxy-Gruppe darstellt,

$R^2$ ein Wasserstoff-Atom oder eine Alkyl-Gruppe mit 1 bis 6 Kohlenstoff-Atomen, eine Phenyl-Gruppe oder eine Benzyl-Gruppe,

$R^3$ ein Wasserstoff-Atom oder eine Alkyl-Gruppe mit 1 bis 4 Kohlenstoff-Atomen,

$R^4$ eine Alkyl-Gruppe mit 1 bis 4 Kohlenstoff-Atomen und

X ein Wasserstoff-Atom, ein Fluor-Atom, ein Chlor-Atom, ein Brom-Atom, eine Methyl-Gruppe oder eine Ethyl-Gruppe.

Die Verbindungen der Formel (I) werden mittels eines Verfahrens erhalten, bei dem

a) in dem Fall, in dem A

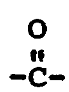

ist,

Verbindungen der Formel (II)

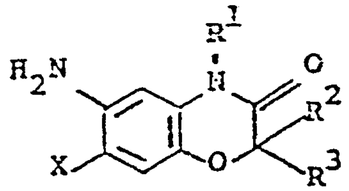

(II)

,

worin $R^1$, $R^2$, $R^3$ und X die oben angegebenen Bedeutungen haben, mit Verbindungen der Formel (III)

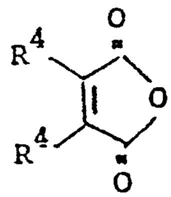

(III)

,

worin $R^4$ die oben angebene Bedeutung hat,
in Gegenwart inerter Lösungsmittel zur Reaktion gebracht werden
oder
b) in dem Fall, in dem A

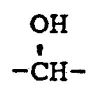

ist, Verbindungen der Formel (I-a)

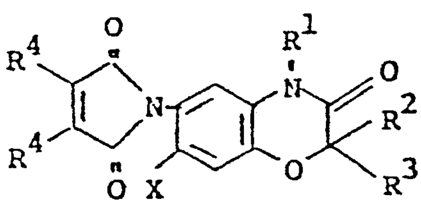

(I-a)

,

worin $R^1$, $R^2$, $R^3$, $R^4$ und X die oben angegebenen Bedeutungen haben, mit einem Reduktionsmittel in Gegenwart inerter Lösungsmittel zur Reaktion gebracht werden
oder
c) in dem Fall, in dem A

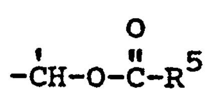

ist,
Verbindungen der Formel (I-b)

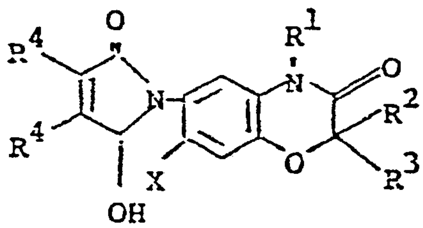

(I-b) ,

worin $R^1$, $R^2$, $R^3$, $R^4$ und X die oben angegebenen Bedeutungen haben, mit Verbindungen der Formel (IV)

$$R^5-\overset{O}{\overset{\|}{C}}-Hal \qquad (IV) ,$$

in der $R^5$ die oben angegebenen Bedeutungen hat und Hai ein Halogen-Atom bezeichnet, oder mit Verbindungen der Formel (V)

$$R^5-\overset{O}{\overset{\|}{C}}-O-\overset{O}{\overset{\|}{C}}-R^5 \qquad (V) ,$$

in der $R^5$ die oben angegebenen Bedeutungen hat, in Gegenwart inerter Lösungsmittel und gegebenenfalls in Gegenwart säurebindender Mittel zur Reaktion gebracht werden.

Die neuen Dialkylmaleinimide zeigen potente herbizide Eigenschaften.

Überraschenderweise zeigen die erfindungsgemäßen Dialkylmaleinimide nicht nur eine wesentlich stärkere herbizide Wirkung als die aus dem oben genannten Stand der Technik bekannten, sondern auch eine günstige Verträglichkeit mit Nutzpflanzen, ohne Phytotoxizität.

Vorzugsweise bezeichnen in der die Verbindungen der vorliegenden Erfindung repräsentierenden Formel (I)

A

$$-\overset{O}{\overset{\|}{C}}-, \quad -\overset{OH}{\overset{|}{CH}}- \text{ oder } -\overset{|}{CH}-O-\overset{O}{\overset{\|}{C}}-R^5,$$

worin $R^5$ eine Methyl-, Ethyl- oder Phenyl-Gruppe darstellt,

$R^1$ ein Wasserstoff-Atom, eine Methyl-, Ethyl-, Propyl-, Allyl-, Propargyl-, Benzyl-, Methoxy-methyl-, Cyanomethyl-, Trimethylsilylmethyl-, Methylthiomethyl- oder 2-Ethylthioethyl-Gruppe oder die Gruppe

$$-\overset{R^6}{\overset{|}{C}}H-COR^7,$$

worin $R^6$ ein Wasserstoff-Atom oder eine Methyl-Gruppe darstellt und $R^7$ eine Methoxy-, Ethoxy-, Cyclopentyloxy-, Cyclohexyloxy-, Trifluoroethoxy-, Isopropylamino-, Dimethylamino-, N-Methyl-N-phenylamino- oder eine Trimethylsilylmethoxy-Gruppe darstellt,

$R^2$ ein Wasserstoff-Atom oder eine Methyl-, Ethyl-, n-Propyl-, n-Butyl-, n-Hexyl-, Phenyl- oder Benzyl-Gruppe

$R^3$ ein Wasserstoff-Atom oder eine Methyl-Gruppe,
$R^4$ eine Methyl-Gruppe und
X ein Wasserstoff- oder ein Fluor-Atom.

Zu speziellen Beispielen für die durch die vorliegende Erfindung verfügbar gemachten Verbindungen der Formel (I) zählen
1-[4-Propyl-2H-1,4-benzoxazin-3(4H)-on-6-yl]-3,4-dimethyl-2,5-dioxo-2,5-dihydropyrrol,
1-[4-Allyl-2H-1,4-benzoxazin-3(4H)-on-6-yl]-3,4-dimethyl-2,5-dioxo-2,5-dihydropyrrol,
1-[4-Propargyl-2H-1,4-benzoxazin-3(4H)-on-6-yl]-3,4-dimethyl-2,5-dioxo-2,5-dihydropyrrol,
1-[4-Propyl-2-methyl-1,4-benzoxazin-3(4H)-on-6-yl]-3,4-dimethyl-2,5-dioxo-2,5-dihydropyrrol und
1-[4-Propyl-2,2-dimethyl-1,4-benzoxazin-3(4H)-on-6-yl]-3,4-dimethyl-2,5-dioxo-2,5-dihydropyrrol.

Wenn in dem Verfahren a) beispielsweise 6-Amino-4-propyl-2H-1,4-benzoxazin-3(4H)-on und 2,3-Dimethylmaleinsäureanhydrid als Ausgangsstoffe eingesetzt werden, wird die Reaktion durch das folgende Schema dargestellt.

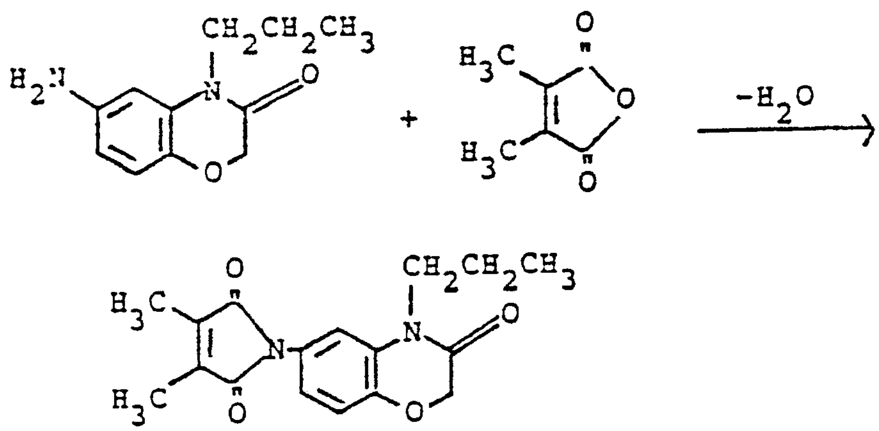


Wenn in dem Verfahren b) beispielsweise 1-[4-Propyl-2H-1,4-benzoxazin-3(4H)-on-6-yl]-3,4-dimethyl-2,5-dioxo-2,5-dihydropyrrol und Natriumborhydrid als Ausgangsstoffe eingesetzt werden, wird die Reaktion durch das folgende Schema dargestellt.

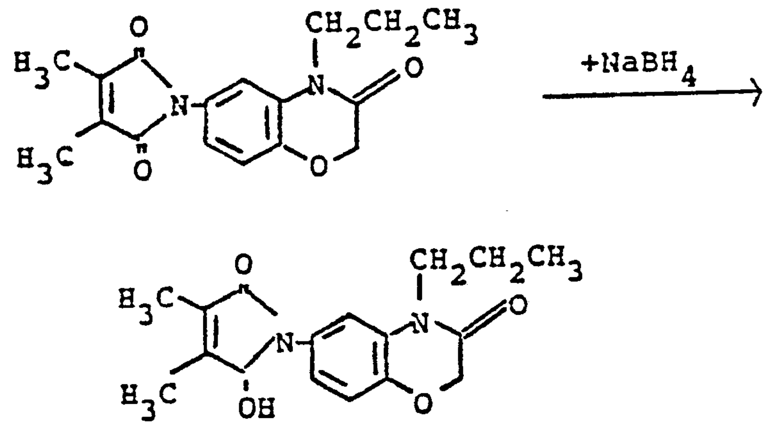


Wenn in dem Verfahren c) beispielsweise 1-[4-Propyl-2H-1,4-benzoxazin-3(4H)-on-6-yl]-3,4-dimethyl-2-hydroxy-5-oxo-2,5-dihydropyrrol und Acetylchlorid als Ausgangsstoffe eingesetzt werden, wird die Reaktion durch das folgende Schema dargestellt.

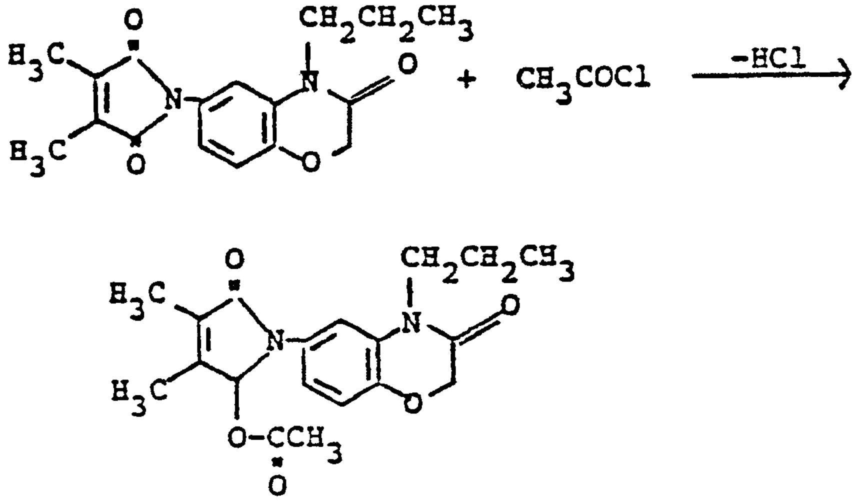


In dem Verfahren a) bezeichnet die Verbindung der Formel (II) als Ausgangsstoff eine auf den obigen Definitionen für $R^1$, $R^2$, $R^3$ und X basierende Verbindung, vorzugsweise eine, die auf den bevorzugten Definitionen dieser Symbole beruht.

Zu den Verbindungen der Formel (II) zählen beispielsweise die bekannten, in den JP-OSen 30586/1986 und 76486/1986 beschriebenen Verbindungen.

Im allgemeinen lassen sich die Verbindungen der Formel (II) in einfacher Weise durch Reduktion des entsprechenden 4-substituierten 6-Nitro-1,4-benzoxazinons nach dem in der oben zitierten JP-OS 30586/1986 beschriebenen Verfahren gewinnen.

Das 4-substituierte 6-Nitro-1,4-benzoxazinon kann ebenfalls in einfacher Weise nach dem Verfahren der JP-OS 30586/1986 hergestellt werden.

Das 4-substituierte 6-Nitro-1,4-benzoxazinon kann in einfacher Weise aus 2-Amino-4-nitrophenol als Ausgangsstoff nach den in der JP-OS 125529/1974 und in "Synthesis" 1984, Seite 851 beschriebenen Arbeitsweisen hergestellt werden.

6-Amino-4-propyl-2H-1,4-benzoxazin-3(4H)-on sei als spezielles Beispiel für die Verbindungen der Formel (II) angeführt.

Die Verbindungen der Formel (III), die in gleicher Weise Ausgangsstoffe sind, bezeichnen auf der obigen Definition für $R^4$ basierende Verbindungen, vorzugsweise solche, die auf den bevorzugten Definitionen für $R^4$ beruhen.

Zu den Verbindungen der Formel (III) zählen beispielsweise die bekannten, in der JP-OS 23965/1978 beschriebenen Verbindungen. Ein spezielles Beispiel ist 2,3-Dimethylmaleinsäureanhydrid.

In dem Verfahren b) zählt die Ausgangsverbindung der Formel (I-a) zu den Verbindungen der Formel (I), die mittels des Verfahrens a) hergestellt werden.

Beispiele für das in dem Verfahren b) eingesetzte Reduktionsmittel sind Metallhydride wie Natriumborhydrid und Lithiumaluminiumhydrid.

Die als Ausgangsstoff in dem Verfahren c) eingesetzte Verbindung der Formel (I-b) zählt zu den Verbindungen der Formel (I) der vorliegenden Erfindung, die mittels des Verfahrens b) hergestellt werden.

Die Verbindungen der Formeln (IV) oder (V), die in gleicher Weise Ausgangsstoffe sind, bezeichnen auf den obigen Definitionen für $R^5$ und Hal basierende Verbindungen, vorzugsweise solche, die auf den bevorzugten Definitionen für $R^5$ und einem Chlor-Atom für Hal beruhen.

Die Verbindungen der Formeln (IV) und (V) sind wohlbekannt, und zu speziellen Beispielen zählen Acetylchlorid und Acetanhydrid.

Für die praktische Durchführung des Verfahrens a) seien sämtliche inerten organischen Lösungsmittel als geeignete Verdünnungsmittel angeführt.

Zu Beispielen für solche Verdünnungsmittel zählen Wasser, halogenierte und aromatische Kohlenwasserstoffe (wie Ethylenchlorid, Chlorobenzol, Dichlorobenzol und Biphenyl), Ether (wie Dioxan und Diphenylether), Alkohole (wie Ethanol, Isopropanol, Butanol und Ethylenglycol) und organische Säuren (wie Essigsäure und Propionsäure).

Das Verfahren a) kann innerhalb eines Temperaturbereichs von beträchtlicher Breite, beispielsweise zwischen etwa 70 °C und etwa 280 °C, vorzugsweise aber zwischen etwa 80 °C und etwa 100 °C, durchgeführt werden.

Zweckmäßigerweise wird die Reaktion unter Atmosphärendruck durchgeführt, jedoch ist es auch möglich, unter erhöhtem oder vermindertem Druck zu arbeiten.

Bei der Durchführung des Verfahrens a) kann beispielsweise 1 mol der Verbindungen der Formel (II) mit etwa 1 bis 1,2 mol der Verbindungen der Formel (III) in einem inerten organischen Lösungsmittel zur Reaktion gebracht werden, um die gewünschten Verbindungen der Formel (I) zu erhalten.

Für die praktische Durchführung des Verfahrens b) seien Wasser, Alkohole (vorzugsweise Methanol) und Ether (vorzugsweise Dioxan und Tetrahydrofuran) als geeignete Verdünnungsmittel angeführt.

Bei der Durchführung des Verfahrens b) können die gewünschten Verbindungen der Formel (I) in einfacher Weise durch Reduktion der Verbindungen der Formel (I-a) mit einem Reduktionsmittel, wie sie oben beispielhaft genannt sind, insbesondere mit Natriumborhydrid, erhalten werden, wie in einem nachstehend angegebenen Arbeitsbeispiel gezeigt wird.

Für die praktische Durchführung des Verfahrens c) seien aromatisohe Kohlenwasserstoffe wie Toluol, Ether wie Diethylether und Tetrahydrofuran, halogenierte Kohlenwasserstoffe wie Dichloromethan und Chloroform sowie Wasser als geeignete Verdünnungsmittel angeführt.

.Das Verfahren c) kann in Gegenwart eines säurebindenden Mittels durchgeführt werden. Beispiele für die säurebindenden Mittel sind Hydride, Hydroxide, Carbonate und Hydrogencarbonate von Alkalimetallen und tertiäre Amine wie Triethylamin, Diethylanilin und Pyridin.

Das Verfahren c) kann innerhalb eines Temperaturbereichs von beträchtlicher Breite, beispielsweise zwischen etwa -10 °C und etwa 100 °C, vorzugsweise zwischen etwa 10 °C und etwa 100 °C, durchgeführt werden.

Zweckmäßigerweise wird die Reaktion unter Atmosphärendruck durchgeführt, jedoch ist es auch möglich, unter erhöhtem oder vermindertem Druck zu arbeiten.

Bei der Durchführung des Verfahrens c) können die gewünschten Verbindungen der Formel (I) beispielsweise durch Umsetzung von 1 mol der Verbindungen der Formel (I-b) mit etwa 1 bis 1,2 mol der Verbindungen der Formel (IV) oder (V) in einem inerten Lösungsmittel, gegebenenfalls in Gegenwart säurebindender Mittel, umgesetzt werden.

Die erfindungsgemäßen aktiven Verbindungen können als Entlaubungsmittel, Abbrandmittel, Mittel zur Zerstörung breitblättriger Pflanzen und insbesondere als Unkrautvernichtungsmittel eingesetzt werden. Unter "Unkräutern" im weitesten Sinne sind alle Pflanzen zu verstehen, die an Stellen wachsen, wo sie nicht erwünscht sind. Ob die erfindungsgemäßen Substanzen als totale oder selektive Herbizide wirken, hängt im wesentlichen von der verwendeten Menge ab.

Die aktiven Verbindungen gemäß der Erfindung können beispielsweise in Verbindung mit den folgenden Pflanzen verwendet werden:

Dikotyledonen-Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver und Centaurea.

Dikotyledonen-Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis und Cucurbita.

Monokotyledonen-Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus und Apera.

Monokotyledonen-Kulturen der Gattungen:

Oryza, Zea, Triticum, Hordeum, Avena, Secale, Sorghum, Panicum, Saccharum, Ananas, Asparagus und Allium.

Die Anwendung der erfindungsgemäßen aktiven Verbindungen ist jedoch keineswegs auf diese Gattungen begrenzt, sondern umfaßt auch andere Pflanzen in gleicher Weise.

In Abhängigkeit von den Konzentrationen können die Verbindungen zur totalen Bekämpfung von Unkräutern, beispielsweise auf Industriegelände und Bahnkörpern sowie auf Wegen und Plätzen mit oder ohne Baumbestand, verwendet werden. Ebenso können die Verbindungen auch zur Unkrautbekämpfung in perennierenden Kulturen, beispielsweise Aufforstungen, Zierbaumpflanzungen, Obstgärten, Weinbergen und -gärten, Zitrushainen, Nußbaumpflanzungen, Bananenplantagen, Kaffeeplantagen, Teeplantagen, Gummiplantagen, Ölpalmenplantagen, Kakaoplantagen, Weichfruchtplantagen und Hopfenfeldern, sowie zur selektiven Unkrautbekämpfung in Jahreskulturen verwendet werden.

Die Wirkstoffe können in übliche Präparate wie Lösungen, Emulsionen, Suspensionen, Pulver, Stäubemittel, Schäume, Pasten, lösliche Pulver, Granulat, Aerosole, mit dem Wirkstoff imprägnierte natürliche und synthetische Materialien, sehr feine Kapseln in polymeren Substanzen, Beschichtungsmittel zum Aufbringen auf das Saatgut und Formulierungen, die in Verbindung mit Verbrennungseinrichtungen wie Räucherpatronen, Räucherdosen und Räucherschlangen eingesetzt werden, sowie ULV-Kaltvernebelungs- und Warmvernebelungspräparate umgewandelt werden.

Diese Präparate können in bekannter Weise hergestellt werden, beispielsweise durch Vermischen der Wirkstoffe mit Streckmitteln, d.h. flüssigen oder verflüssigten gasförmigen oder festen Verdünnungsmitteln oder Trägern, gegebenenfalls unter Verwendung von grenzflächenaktiven Mitteln, d.h. Emulgatoren und/oder Dispergiermitteln und/oder schaumbildenden Mitteln. Bei Verwendung von Wasser als Streckmittel können beispielsweise auch organische Lösungsmittel als Hilfslösungsmittel verwendet werden.

Als flüssige Verdünnungsmittel oder Träger eignen sich hauptsächlich aromatische Kohlenwasserstoffe wie Xylol, Toluol oder Alkylnaphthaline, chlorierte aromatische oder chlorierte aliphatische Kohlenwasserstoffe wie Chlorobenzole, Chloroethylene oder Methylenchlorid, aliphatische oder alicyclische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon, oder stark polare Lösungsmittel wie Dimethylformamid oder Dimethylsulfoxid, sowie auch Wasser.

Unter verflüssigten gasförmigen Verdünnungsmitteln oder Trägern sind Flüssigkeiten zu verstehen, die bei normaler Temperatur und unter normalem Druck gasförmig sein würden, beispielsweise Aerosol-Treibmittel wie halogenierte Kohlenwasserstoffe sowie Butan, Propan, Stickstoff und Kohlenstoffdioxid.

Als feste Träger können gemahlene natürliche Minerale wie Kaoline, Tone, Talkum, Kreide, Quarz, Attapulgit, Montmorillonit oder Diatomeenerde, und gemahlene synthetische Minerale wie hochdisperse Kieselsäure, Aluminiumoxid und Silikate verwendet werden. Als feste Träger für Granulat können zerkleinertes und fraktioniertes natürliches Gesteinsmaterial wie Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengeln verwendet werden.

Als Emulgatoren und/oder Schaumbildner können nichtionische oder anionische Emulgatoren wie Polyoxyethylen-Fettsäureester, Polyoxyethylen-Fettalkoholether, beispielsweise Alkylarylpolyglycolether, Alkylsulfonate, Alkylsulfate, Arylsulfonate sowie Hydrolyseprodukte des Albumins verwendet werden. Zu den Dispergiermitteln zählen beispielsweise Ligninsulfit-Ablaugen und Methylcellulose.

Haftmittel wie Carboxymethylcellulose und natürliche und synthetische Polymerisate in Form von Pulver, Granulat oder Latices, z.B. Gummi arabicum, Polyvinylalkohol und Polyvinylacetat, können in den Formulierungen verwendet werden.

Es ist möglich, farbgebende Stoffe wie anorganische Pigmente, beispielsweise Eisenoxid, Titanoxid und Preußischblau, und organische Farbstoffe wie Alizarin-Farbstoffe, Azo-Farbstoffe oder Metallphthalocyanin-Farbstoffe, und Spurennährstoffe wie Salze von Eisen, Mangan, Bor, Kupfer, Cobalt, Molybdän und Zink, zu verwenden.

Die Präparate enthalten im allgemeinen von 0,1 bis 95 Gewichts-%, vorzugsweise von 0,5 bis 90 Gewichts-% des Wirkstoffs.

Die Wirkstoffe gemäß der vorliegenden Erfindung können als solche oder in Form ihrer Präparate zur Unkrautbekämpfung als Mischungen mit anderen Herbiziden eingesetzt werden, wobei Fertigpräparate oder Tankmischungen möglich sind.

Mischungen mit anderen aktiven Verbindungen wie Herbiziden, Fungiziden, Insektiziden, Akariziden, Nematiziden, vogelabweisenden Mitteln, Pflanzennährstoffen sowie Mitteln zur Verbesserung der Bodenstruktur sind ebenfalls möglich.

Die Wirkstoffe können als solche, in Form von Präparaten oder in Gebrauchsformen, die daraus durch weitere Verdünnung hergestellt werden, eingesetzt werden, beispielsweise in Form gebrauchsfertiger Lösungen, Suspensionen, Emulsionen, Pulver, Pasten und als Granulat. Sie werden in üblicher Weise angewandt, beispielsweise durch Bewässern, Sprühen, Zerstäuben oder Streuen.

Die erfindungsgemäßen Wirkstoffe können vor oder nach dem Auflaufen der Pflanzen zur Anwendung gelangen.

Sie können auch vor der Einsaat in den Boden eingearbeitet werden. Insbesondere werden sie nach dem Auflaufen der Pflanzen angewandt.

Die flächenbezogenen Aufwandsmengen der Aktiven Verbindung können innerhalb eines Bereichs von erheblicher Breite variiert werden. Sie hängen wesentlich von der Art der angestrebten Wirkung ab. Im allgemeinen liegen die aufgewandten Mengen des Wirkstoffs, bezogen auf die Fläche des Bodens, zwischen etwa 0,001 und etwa 5 kg/ha, vorzugsweise zwischen etwa 0,01 und etwa 2,5 kg/ha.

Die Herstellung und Verwendung der erfindungsgemäßen Verbindungen sind den folgenden Beispielen zu entnehmen.

Herstellungsbeispiele

BEISPIEL 1

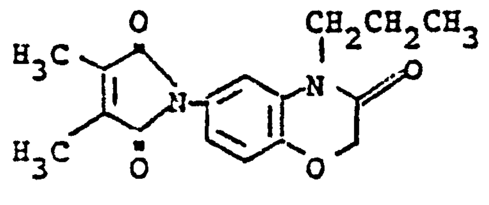

(Verbindung Nr. 1)

2,27 g 6-Amino-4-propyl-2H-1,4-benzoxazin-3(4H)-on und 1,7 g 2,3-Dimethylmaleinsäureanhydrid wurden in Essigsäure 2 h unter Rückfluß erhitzt. Man ließ die Reaktionsmischung abkühlen. Nach Zusatz von 50 ml Wasser wurden die ausgefallenen Kristalle durch Filtration gesammelt und aus Ethanol umkristallisiert, wonach 1,7 g 1-[4-Propyl-2H-1,4-benzoxazin-3(4H)-on-6-yl]-3,4-dimethyl-2,5-dioxo-2,5-dihydropyrrol erhalten wurden; Schmp. 147-149 °C.

BEISPIEL 2

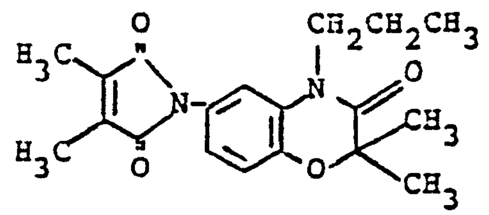

(Verbindung Nr. 9)

1,83 g 6-Amino-4-propyl-2,2-dimethyl-1,4-benzoxazin-3(4H)-on und 0,96 g 2,3-Dimethylmaleinsäureanhydrid wurden in 10 ml Essigsäure 2 h unter Rückfluß erhitzt. Man ließ die Reaktionsmischung abkühlen. Nach Zusatz von 50 ml Wasser wurden die ausgefallenen Kristalle durch Filtration gesammelt und aus Ethanol umkristallisiert, wonach 1,31 g 1-[4-Propyl-2,2-dimethyl-1,4-benzoxazin-3(4H)-on-6-yl]-3,4-dimethyl-2,5-dioxo-2,5-dihydropyrrol erhalten wurden; Schmp. 120-121 °C.

BEISPIEL 3

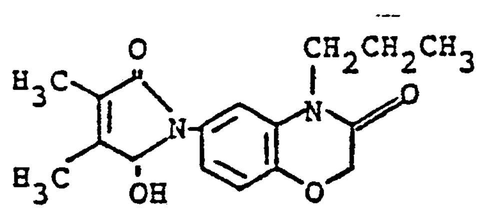

(Verbindung Nr. 33)

1,15 g 1-[4-Propyl-2H-1,4-benzoxazin-3(4H)-on-6-yl]-3,4-dimethyl-2,5-dioxo-2,5-dihydropyrrol wurden in 100 ml Methanol gelöst, und 0,09 g Natriumborhydrid wurden bei Raumtemperatur hinzugefügt. Die Mischung wurde bei dieser Temperatur 3 h gerührt. Ein Tropfen Essigsäure wurde zugegeben, und das Methanol wurde abgedampft. Der konzentrierte Rückstand wurde mit Dichloromethan extrahiert, mit Wasser gewaschen und getrocknet. Das Lösungsmittel wurde abgedampft, und die ausgefallenen Kristalle wurden aus Ethanol umkristallisiert, wonach 0,8 g 1-[4-Propyl-2H-1,4-benzoxazin-3(4H)-on-6-yl]-3,4-dimethyl-2-hydroxy-5-oxo-2,5-dihydropyrrol erhalten wurden; Schmp. 124-125 °C.

Die Verbindungen der Formel (I) gemäß der vorliegenden Erfindung können mittels der gleichen Arbeitsweisen wie in den Beispielen 1 bis 3 hergestellt werden und sind zusammen mit den in den Beispielen 1 bis 3 erhaltenen Verbindungen in Tabelle 1 aufgeführt.

## Tabelle 1

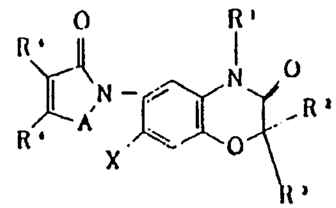

| Verb. Nr. | A | $R^1$ | $R^2$ | $R^3$ | $R^4$ | X | Schmp. °C |
|---|---|---|---|---|---|---|---|
| 1 | $-\overset{O}{\overset{\|}{C}}-$ | $-C_3H_7-n$ | H | H | $-CH_3$ | H | 147 ~ 149 |
| 2 | $-\overset{O}{\overset{\|}{C}}-$ | $-C_3H_7-n$ | $-CH_3$ | H | $-CH_3$ | H | 146 ~ 148 |
| 3 | $-\overset{O}{\overset{\|}{C}}-$ | $-C_3H_7-n$ | $-C_2H_5$ | H | $-CH_3$ | H | 143 ~ 144 |
| 4 | $-\overset{O}{\overset{\|}{C}}-$ | $-C_3H_7-n$ | $-C_3H_7-n$ | H | $-CH_3$ | H | 117.5 ~ 119.5 |
| 5 | $-\overset{O}{\overset{\|}{C}}-$ | $-C_3H_7-n$ | $-C_4H_9-n$ | H | $-CH_3$ | H | 86.5 ~ 88.5 |

| Verb. Nr. | A | $R^1$ | $R^2$ | $R^3$ | $R^4$ | X | Schmp. °C |
|---|---|---|---|---|---|---|---|
| 6 | $-\overset{O}{\overset{\|}{C}}-$ | $-C_3H_7-n$ | $-C_6H_{13}-n$ | H | $-CH_3$ | H | 95～96 |
| 7 | $-\overset{O}{\overset{\|}{C}}-$ | $-C_3H_7-n$ | 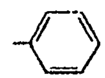 | H | $-CH_3$ | H | 190～192 |
| 8 | $-\overset{O}{\overset{\|}{C}}-$ | $-C_3H_7-n$ | $-CH_2$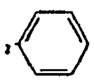 | H | $-CH_3$ | H | 120～122 |
| 9 | $-\overset{O}{\overset{\|}{C}}-$ | $-C_3H_7-n$ | $-CH_3$ | $-CH_3$ | $-CH_3$ | H | 120～121 |
| 10 | $-\overset{O}{\overset{\|}{C}}-$ | $-CH_3$ | H | H | $-CH_3$ | H | 226～228 |
| 11 | $-\overset{O}{\overset{\|}{C}}-$ | $-C_2H_5$ | H | H | $-CH_3$ | H | 212.5～213.5 |
| 12 | $-\overset{O}{\overset{\|}{C}}-$ | $-C_4H_9-n$ | H | H | $-CH_3$ | H | 142～144 |

| Verb. Nr. | A | $R^1$ | $R^2$ | $R^3$ | $R^4$ | X | Schmp. °C |
|---|---|---|---|---|---|---|---|
| 13 | $-\overset{O}{\overset{\|}{C}}-$ | $-CH_2-CH=CH_2$ | H | H | $-CH_3$ | H | 199~200 |
| 14 | $-\overset{O}{\overset{\|}{C}}-$ | $-CH_2-C\equiv CH$ | H | H | $-CH_3$ | H | 268~270 |
| 15 | $-\overset{O}{\overset{\|}{C}}-$ | $-CH_2CN$ | H | H | $-CH_3$ | H | 168~171 |
| 16 | $-\overset{O}{\overset{\|}{C}}-$ | $-CH_2OCH_3$ | H | H | $-CH_3$ | H | 195~198 |
| 17 | $-\overset{O}{\overset{\|}{C}}-$ | $-CH_2$ 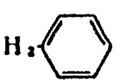 | H | H | $-CH_3$ | H | 176.5~178 |
| 18 | $-\overset{O}{\overset{\|}{C}}-$ | $-CH_2COOC_2H_5$ | H | H | $-CH_3$ | H | 129~132 |
| 19 | $-\overset{O}{\overset{\|}{C}}-$ | $-CH_2COO-$ 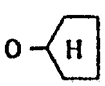 | H | H | $-CH_3$ | H | 106~108 |

| Verb. Nr. | A | R¹ | R² | R³ | R⁴ | X | Schmp. °C |
|---|---|---|---|---|---|---|---|
| 20 | 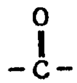 | $-CH-CON(CH_3)_2$ | H | H | $-CH_3$ | H | 176 - 179 |
| 21 | 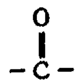 | 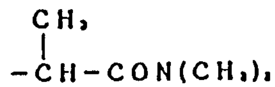 | H | H | $-CH_3$ | H | 180~181.5 |
| 22 | 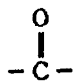 | $-CH_2CONHCH(CH_3)_2$ | H | H | $-CH_3$ | H | 282 - 290 |
| 23 | 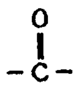 | 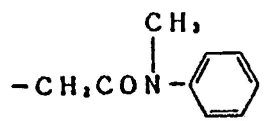 | H | H | $-CH_3$ | H | 177~178 |
| 24 | 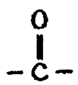 | $-CH_2SCH_3$ | H | H | $-CH_3$ | H | 196 - 199 |
| 25 | 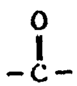 | $-CH_2CH_2SC_2H_5$ | H | H | $-CH_3$ | H | 142 - 144 |
| 26 | 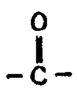 | $-C_3H_7-n$ | H | H | $-C_2H_5$ | H | 132~135 |

| Verb. Nr. | A | $R^1$ | $R^2$ | $R^3$ | $R^4$ | X | Schmp. °C |
|---|---|---|---|---|---|---|---|
| 27 | 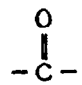 | $-C_3H_7-n$ | H | H | $-CH_3$ | $-CH_3$ | 164 - 165 |
| 28 | 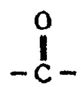 | $-C_3H_7-n$ | H | H | $-CH_3$ | F | 112 - 113 |
| 29 | 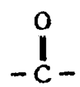 | $-C_3H_7-n$ | $-CH_3$ | H | $-CH_3$ | F | |
| 30 | 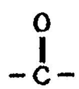 | $-C_3H_7-n$ | H | H | $-CH_3$ | Cl | 122 - 123 |
| 31 | 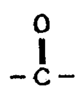 | $-CH_2OC_2H_5$ | H | H | $-CH_3$ | H | |
| 32 | 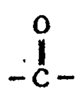 | $-CH_2COOCH_2CF_3$ | H | H | $-CH_3$ | H | 130∼137 |
| 33 | 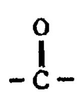 | $-CH_2SC_2H_5$ | H | H | $-CH_3$ | H | 197 - 201 |

| Verb. Nr. | A | $R^1$ | $R^2$ | $R^3$ | $R^4$ | X | Schmp. °C |
|---|---|---|---|---|---|---|---|
| 34 | 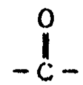 | $-CH_2C\equiv CH$ | H | H | $-CH_3$ | F | 240 - 241 |
| 35 | 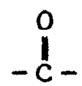 | $-CH_2C\equiv CH$ | $-CH_3$ | H | $-CH_3$ | F | |
| 36 | 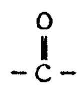 | $-CH_2COOC_2H_5$ | H | H | $-CH_3$ | F | |
| 37 | 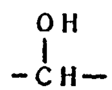 | $-C_3H_7-n$ | H | H | $-CH_3$ | H | 124～125 |
| 38 | 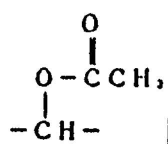 | $-C_3H_7-n$ | H | H | $-CH_3$ | H | 137～140 |
| 39 | 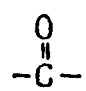 | $-CH_2COOCH_2Si(CH_3)_3$ | H | H | $-CH_3$ | H | 113～117 |

| Verb. Nr. | A | $R^1$ | $R^2$ | $R^3$ | $R^4$ | X | Schmp. °C |
|---|---|---|---|---|---|---|---|
| 40 | $-\overset{\overset{O}{\|}}{C}-$ | $-CH_2OCH_3$ | H | H | $-CH_3$ | F | 153 - 154 |
| 41 | $-\overset{\overset{O}{\|}}{C}-$ | $-CH_2OC_2H_5$ | H | H | $-CH_3$ | F | |
| 42 | $-\overset{\overset{O}{\|}}{C}-$ | $-CH_2OC_2H_5$ | H | H | $-CH_3$ | H | 136 - 137.5 |
| 43 | $-\overset{\overset{O}{\|}}{C}-$ | $-CH_2CH{=}CH_2$ | H | H | $-CH_3$ | F | 165 - 166.5 |
| 44 | $-\overset{\overset{O}{\|}}{C}-$ | $-CH_2CN$ | H | H | $-CH_3$ | F | 236 - 239 |
| 45 | $-\overset{\overset{O}{\|}}{C}-$ | $-CH_2-COOCH_2Si(CH_3)_3$ | H | H | $-CH_3$ | F | 123 - 125 |
| 46 | $-\overset{\overset{O}{\|}}{C}-$ | H | H | H | $-CH_3$ | H | 238 - 242 |

| Verb. Nr. | A | $R^1$ | $R^2$ | $R^3$ | $R^4$ | X | Schmp. °C |
|---|---|---|---|---|---|---|---|
| 47 | $-\overset{O}{\overset{\parallel}{C}}-$ | $-CH_2-C\equiv CH$ | H | H | $-CH_3$ | $-CH_3$ | 255 - 256.5 |
| 48 | $-\overset{O}{\overset{\parallel}{C}}-$ | $-CH_2-C\equiv CH$ | H | H | $-CH_3$ | -Cl | 249 - 251 |
| 49 | $-\overset{O}{\overset{\parallel}{C}}-$ | $-CH_2-CH=CH-CH_3$ | H | H | $-CH_3$ | H | 146 - 147.5 |
| 50 | $-\overset{O}{\overset{\parallel}{C}}-$ | $-\underset{CH_3}{\underset{\mid}{CH}}-CH=CH_2$ | H | H | $-CH_3$ | H | 106 - 110 |
| 51 | $-\overset{O}{\overset{\parallel}{C}}-$ | $-CH_2-\underset{CH_3}{\underset{\mid}{C}}=CH_2$ | H | H | $-CH_3$ | H | 107 - 123 |
| 52 | $-\overset{O}{\overset{\parallel}{C}}-$ | $-CH_2-CH_2-CH=CH_2$ | H | H | $-CH_3$ | H | 122 - 133 |
| 53 | $-\overset{O}{\overset{\parallel}{C}}-$ | $-\underset{CH_3}{\underset{\mid}{CH}}-CN$ | H | H | $-CH_3$ | H | 142 - 145 |

| Verb. Nr. | A | $R^1$ | $R^2$ | $R^3$ | $R^4$ | X | Schmp. °C |
|---|---|---|---|---|---|---|---|
| 54 | $-\overset{O}{\overset{\|}{C}}-$ | $-CH_2-CH_2-O-C_2H_5$ | H | H | $-CH_3$ | H | 118 - 119 |
| 55 | $-\overset{O}{\overset{\|}{C}}-$ | $-CH_2-O-CH_2-C{\equiv}CH$ | H | H | $-CH_3$ | H | 185 - 190 |
| 56 | $-\overset{O}{\overset{\|}{C}}-$ | $-C_3H_7$-n | 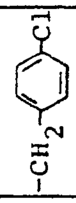 | H | $-CH_3$ | H | 109 - 111 |
| 57 | $-\overset{O}{\overset{\|}{C}}-$ | $-CH_2-CH=CH_2$ | 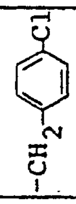 | H | $-CH_3$ | H | 92 - 102 |
| 58 | $-\overset{O}{\overset{\|}{C}}-$ | $-CH_2-C{\equiv}CH$ | 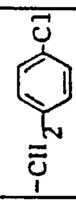 | H | $-CH_3$ | H | 115 - 125 |

Biologische Tests

(Vergleichs-Verbindung E-1)

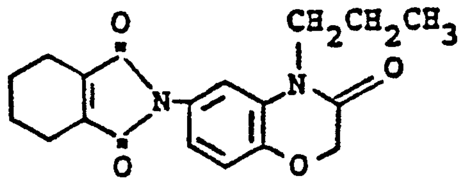

(die in der JP-OS 30586/1986 beschriebene Verbindung).

BEISPIEL 4

Test gegen Unkräuter in einem überfluteten Reisfeld durch Wasseroberflächen-Anwendung

Herstellung eines Wirkstoff-Präparats

Träger: 5 Gewichtsteile Aceton;
Emulgator: 1 Gewichtsteil Benzyloxypolyglycolether.

Ein Wirkstoff-Präparat in Form eines emulgierbaren Konzentrats wurde erhalten durch Vermischen von 1 Gewichtsteil der aktiven Verbindung mit den oben angegebenen Mengen des Trägers und des emulgierenden Mittels. Eine vorher festgelegte Menge des Präparats wurde mit Wasser verdünnt.

Test-Verfahren

Reis-Kulturboden wurde in Töpfe (5 $dm^2$; 25 cm x 20 cm x 9 cm) gefüllt, und Reis-Setzlinge (Varietät: "Nihonbare") im 2,5-Blattstadium (15 cm hoch) wurden jeweils an zwei Stellen pro Topf als Stock von drei Setzlingen umgepflanzt. Samen von Hühnerhirse (Echinochloa oryzicola Vasing.), Schirmkraut (Cyperus difformis L.), Monochoria (Monochoria vaginalis) sowie einjährigen breitblättrigen Unkräutern [Falsche Pimpernelle (Lindernia pyxidaria L.), Rotala indica, Amerikanischer Wasserwurz (Elatine triandra), Rotstengel (Ammannia multiflora Roxburgh) und Dopatrium junceum Hamilton] wurden eingesät und die Töpfe wurden feucht gehalten. Zwei Tage später wurden die Töpfe bis zu einer Tiefe von etwa 2 bis 3 cm überflutet. Fünf Tage nach dem Umpflanzen der Setzlinge wurde die Verbindung der vorliegenden Erfindung, in Form eines wie im Vorstehenden hergestellten emulgierbaren Konzentrats mittels einer Pipette in einer vorher festgelegten Menge auf die Oberfläche des Wassers aufgebracht. Danach wurde der Zustand der etwa 3 cm tiefen Überflutung aufrechterhalten, und vier Wochen nach der chemischen Behandlung wurden die herbizide Wirkung und die Phytotoxizität gegenüber Reis untersucht und mit Hilfe der folgenden Skala von 0 bis 5 bewertet:

| Bewertung | Herbizide Wirkung (bewertet als Unkrautvernichtungs-Verhältnis, bezogen auf eine unbehandelte Fläche) |
|---|---|
| 5 | wenigstens 95 % (verdorrt) |
| 4 | wenigstens 80 %, jedoch weniger als 95 % |
| 3 | wenigstens 50 %, jedoch weniger als 80 % |
| 2 | wenigstens 30 %, jedoch weniger als 50 % |
| 1 | wenigstens 10 %, jedoch weniger als 30 % |
| 0 | weniger als 10 % (unwirksam) |

| Bewertung | Phytotoxizität gegenüber Reis (bewertet unter Bezug auf die unbehandelte Fläche) |
|---|---|
| 5 | wenigstens 90 % (Ausrottung) |
| 4 | wenigstens 50 %, jedoch weniger als 90 % |
| 3 | wenigstens 30 %, jedoch weniger als 50 % |
| 2 | wenigstens 10 %, jedoch weniger als 30 % |
| 1 | mehr als 0 %, jedoch weniger als 10 % |
| 0 | 0 % (keine Phytotoxizität) |

Die Test-Ergebnisse sind anhand typischer Beispiele in Tabelle 2 aufgeführt.

Tabelle 2

| Verbindung Nr. | Wirkstoff-Menge kg/ha | Herbizide Wirkung Hühnerhirse | Schirmkraut | Monochoria | Breitblättrige Unkräuter | Phytotoxizität gegen Reis |
|---|---|---|---|---|---|---|
| 1 | 0,5 | 5 | 5 | 5 | 5 | 1 |
| | 0,25 | 4 | 5 | 5 | 5 | 0 |
| | 0,125 | 4 | 5 | 4 | 4 | 0 |
| Vergleichs-Verbindung | | | | | | |
| E-1 | 0,5 | 5 | 5 | 5 | 5 | 4 |
| | 0,25 | 3 | 5 | 4 | 4 | 3 |
| | 0,125 | 1 | 4 | 2 | 2 | 2 |

BEISPIEL 5

Test gegen Unkräuter auf höher gelegenem Ackerboden durch Behandlung vor dem Auflaufen:
In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), grauem Amaranth (Amaranthus lividus L.) und Gänsefuß (Chenopodium album L.) enthielt, wurde über die Erde in den Töpfen in einer Tiefe von 1 cm gestreut.

Einen Tag nach der Einsaat wurde eine Test-Chemikalie in einer vorher festgelegten Konzentration, hergestellt wie in Beispiel 4, gleichmäßig über die Oberflächenschicht des Bodens in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 4 geprüft. Die Ergebnisse sind anhand typischer Beispiele in Tabelle 3 aufgeführt.

Tabelle 3

| Verbindung | Wirkstoff-Menge | Herbizide Wirkung | | | Phytotoxizität gegen Sojabohnen |
|---|---|---|---|---|---|
| | | Hühnerhirse | Grauer Amaranth | Gänsefuß | |
| Nr. | kg/ha | | | | |
| 1 | 0,5 | 5 | 5 | 5 | 1 |
| | 0,25 | 4 | 5 | 5 | 0 |
| | 0,125 | 3 | 5 | 5 | 0 |
| Vergleichs-Verbindung | | | | | |
| E-1 | 0,5 | 4 | 5 | 5 | 4 |
| | 0,25 | 3 | 5 | 5 | 3 |
| | 0,125 | 3 | 4 | 3 | 3 |

BEISPIEL 6

Test gegen Unkräuter auf höher gelegenem Ackerboden durch Laub-Behandlung:
In einem Gewächshaus wurden Sojabohnen-Samen in Töpfe von 500 $cm^2$ Fläche, die mit Ackererde gefüllt waren, eingesät, und Erde, die Samen von Hühnerhirse (Echinocloa crus-galli), grauem Amaranth (Amaranthus lividus L.) und Gänsefuß (Chenopodium album L.) enthielt, wurde über die Erde in den Töpfen in einer Tiefe von 1 cm gestreut.

Nach der Einsaat wurden die Pflanzen 14 Tage angezogen, und eine Test-Chemikalie in einer vorher festgelegten Konzentration, hergestellt wie in Beispiel 4, wurde gleichmäßig über Test-Pflanzen in jedem der Test-Töpfe gesprüht.

Vier Wochen nach dem Sprühen wurden die herbizide Wirkung und die Phytotoxizität gegenüber den Nutzpflanzen anhand der gleichen Bewertungsskalen wie in Beispiel 4 geprüft. Die Ergebnisse sind anhand typischer Beispiele in Tabelle 4 aufgeführt.

Tabelle 4

| Verbindung Nr. | Wirkstoff-Menge kg/ha | Herbizide Wirkung: Hühnerhirse | Herbizide Wirkung: Grauer Amaranth | Herbizide Wirkung: Gänsefuß | Phytotoxizität gegen Sojabohnen |
|---|---|---|---|---|---|
| 1 | 0,5 | 5 | 5 | 5 | 2 |
| | 0,25 | 5 | 5 | 5 | 1 |
| | 0,125 | 4 | 5 | 5 | 0 |
| Vergleichs-Verbindung | | | | | |
| E-1 | 0,5 | 5 | 5 | 5 | 5 |
| | 0,25 | 5 | 5 | 5 | 4 |
| | 0,125 | 4 | 5 | 5 | 3 |

## Patentansprüche

1. Dialkylmaleinimide der Formel (I)

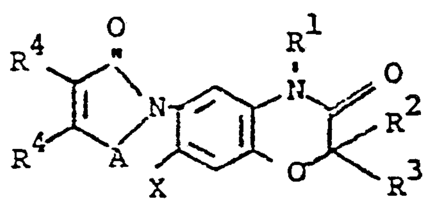

(I)

in der

A

$$-\overset{O}{\overset{\|}{C}}-,\quad -\overset{OH}{\overset{|}{C}H}-\ \text{oder}\ -\overset{|}{C}H-O-\overset{O}{\overset{\|}{C}}-R^5,$$

worin $R^5$ eine Alkyl-Gruppe mit 1 bis 4 Kohlenstoff-Atomen oder eine Phenyl-Gruppe darstellt,

$R^1$ ein Wasserstoff-Atom, eine Alkyl-Gruppe mit 1 bis 4 Kohlenstoff-Atomen, eine Alkenyl-Gruppe mit 2 bis 4 Kohlenstoff-Atomen, eine Alkinyl-Gruppe mit 2 bis 4 Kohlenstoff-Atomen, eine Benzyl-Gruppe, eine Alkoxyalkyl-Gruppe mit insgesamt 2 bis 6 Kohlenstoff-Atomen, eine Cyanomethyl-Gruppe, eine Trimethylsilylmethyl-Gruppe, eine Alkylthioalkyl-Gruppe mit insgesamt 2 bis 6 Kohlenstoff-Atomen oder die Gruppe

$$-\overset{R^6}{\overset{|}{C}}H-COR^7,$$

worin $R^6$ ein Wasserstoff-Atom oder eine Alkyl-Gruppe mit 1 bis 2 Kohlenstoff-Atomen darstellt und $R^7$ eine Alkoxy-Gruppe mit 1 bis 4 Kohlenstoff-Atomen, eine Cycloalkoxy-Gruppe mit 3 bis 7 Kohlenstoff-Atomen, eine Halogenoalkoxy-Gruppe mit 1 bis 4 Kohlenstoff-Atomen, eine Alkylaminooder Dialkylamino-Gruppe mit insgesamt 2 bis 6 Kohlenstoff-Atomen, eine N-($C_1$-$C_4$) Alkyl-N-phenylamino-Gruppe oder eine Trimethylsilylmethoxy-Gruppe darstellt,

$R^2$ ein Wasserstoff-Atom oder eine Alkyl-Gruppe mit 1 bis 6 Kohlenstoff-Atomen, eine Phenyl-Gruppe oder eine Benzyl-Gruppe,

$R^3$ ein Wasserstoff-Atom oder eine Alkyl-Gruppe mit 1 bis 4 Kohlenstoff-Atomen,

$R^4$ eine Alkyl-Gruppe mit 1 bis 4 Kohlenstoff-Atomen und

X ein Wasserstoff-Atom, ein Fluor-Atom, ein Chlor-Atom, ein Brom-Atom, eine Methyl-Gruppe oder eine Ethyl-Gruppe

bezeichnen.

**2.** Verbindungen nach Anspruch 1, dadurch gekennzeichnet, daß

A

$$-\overset{O}{\overset{\|}{C}}-,\quad -\overset{OH}{\overset{|}{C}}H-\ \text{oder}\ -\overset{|}{C}H-O-\overset{O}{\overset{\|}{C}}-R^5,$$

worin $R^5$ eine Methyl-, Ethyl- oder Phenyl-Gruppe darstellt,

$R^1$ ein Wasserstoff-Atom, eine Methyl-, Ethyl-, Propyl-, Allyl-, Propargyl-, Benzyl-, Methoxymethyl-, Cyanomethyl-, Trimethylsilylmethyl-, Methylthiomethyl- oder 2-Ethylthioethyl-Gruppe oder die Gruppe

$$-\overset{R^6}{\overset{|}{C}}H-COR^7,$$

worin $R^6$ ein Wasserstoff-Atom oder eine Methyl-Gruppe darstellt und $R^7$ eine Methoxy-, Ethoxy-, Cyclopentyloxy-, Cyclohexyloxy-, Trifluoroethoxy-, Isopropylamino-, Dimethylamino-, N-Methyl-N-phenylamino- oder eine Trimethylsilylmethoxy-Gruppe darstellt,

$R^2$ ein Wasserstoff-Atom oder eine Methyl-, Ethyl-, n-Propyl-, n-Butyl-, n-Hexyl-, Phenyl- oder Benzyl-Gruppe

$R^3$ ein Wasserstoff-Atom oder eine Methyl-Gruppe,

$R^4$ eine Methyl-Gruppe und

X ein Wasserstoff- oder ein Fluor-Atom

bezeichnen.

**3.** Verbindungen gemäß Anspruch 1, bezeichnet durch eine der Formeln

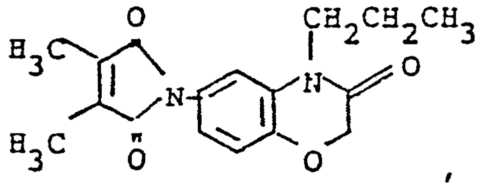

,

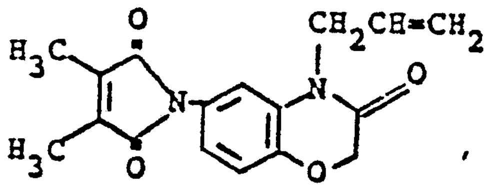

,

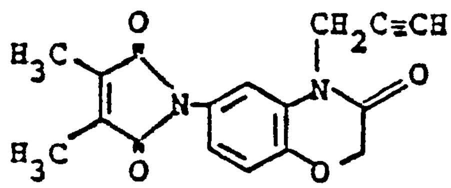

,

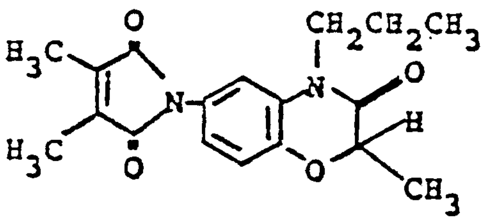

oder

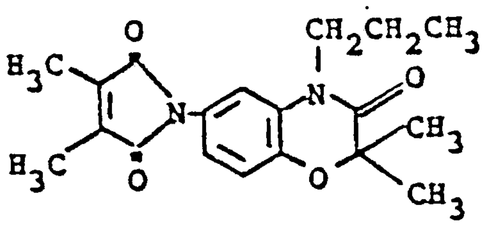

4. Dialkylmaleinimide, bezeichnet durch eine der Formeln

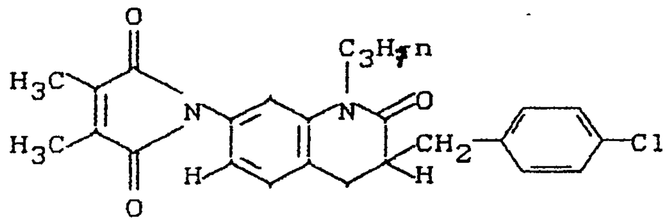

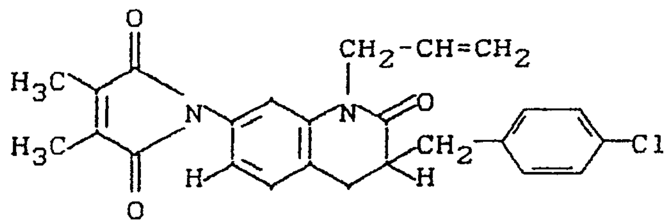

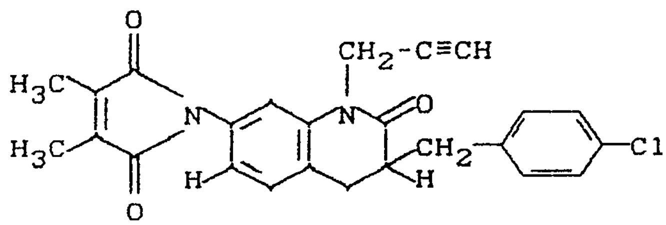

5. Verfahren zur Herstellung von Dialkylmaleinimiden der Formel (I)

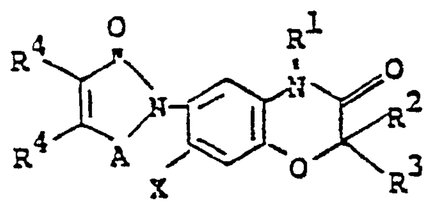 (I)

,

in der

A

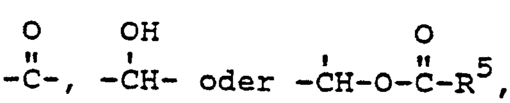

worin $R^5$ eine Alkyl-Gruppe mit 1 bis 4 Kohlenstoff-Atomen oder eine Phenyl-Gruppe darstellt,

$R^1$ ein Wasserstoff-Atom, eine Alkyl-Gruppe mit 1 bis 4 Kohlenstoff-Atomen, eine Alkenyl-Gruppe mit 2 bis 4 Kohlenstoff-Atomen, eine Alkinyl-Gruppe mit 2 bis 4 Kohlenstoff-Atomen, eine Benzyl-Gruppe, eine Alkoxyalkyl-Gruppe mit insgesamt 2 bis 6 Kohlenstoff-Atomen, eine Cyanomethyl-Gruppe, eine Trimethylsilylmethyl-Gruppe, eine Alkylthioalkyl-Gruppe mit insgesamt 2 bis 6 Kohlenstoff-Atomen oder die Gruppe

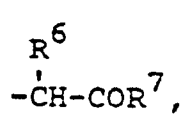

worin $R^6$ ein Wasserstoff-Atom oder eine Alkyl-Gruppe mit 1 bis 2 Kohlenstoff-Atomen darstellt und $R^7$ eine Alkoxy-Gruppe mit 1 bis 4 Kohlenstoff-Atomen, eine Cycloalkoxy-Gruppe mit 3 bis 7 Kohlenstoff-Atomen, eine Halogenoalkoxy-Gruppe mit 1 bis 4 Kohlenstoff-Atomen, eine Alkylaminooder Dialkylamino-Gruppe mit insgesamt 2 bis 6 Kohlenstoff-Atomen, eine N-($C_1$-$C_4$) Alkyl-N-phenylamino-Gruppe oder eine Trimethylsilylmethoxy-Gruppe darstellt,

$R^2$ ein Wasserstoff-Atom oder eine Alkyl Gruppe mit 1 bis 6 Kohlenstoff-Atomen, eine Phenyl-Gruppe oder eine Benzyl-Gruppe,

$R^3$ ein Wasserstoff-Atom oder eine Alkyl-Gruppe mit 1 bis 4 Kohlenstoff-Atomen,

$R^4$ eine Alkyl-Gruppe mit 1 bis 4 Kohlenstoff-Atomen und

X ein Wasserstoff-Atom, ein Fluor-Atom, ein Chlor-Atom, ein Brom-Atom, eine Methyl-Gruppe oder eine Ethyl-Gruppe

bezeichnen,

dadurch gekennzeichnet, daß

a) in dem Fall, in dem A

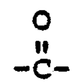

ist,

Verbindungen der Formel (II)

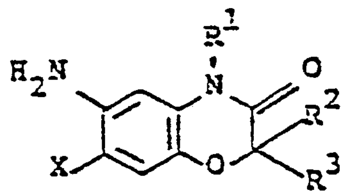

(II)

,

worin $R^1$, $R^2$, $R^3$ und X die oben angegebenen Bedeutungen haben, mit Verbindungen der Formel (III)

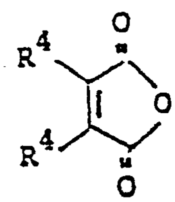

(III)

,

worin $R^4$ die oben angebene Bedeutung hat,
in Gegenwart inerter Lösungsmittel zur Reaktion gebracht werden
oder

b) in dem Fall, in dem A

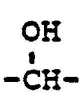

ist,

Verbindungen der Formel (I-a)

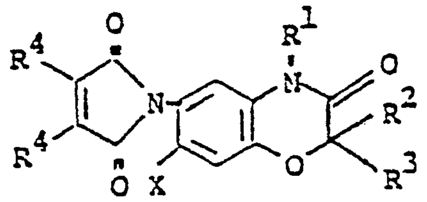

(I-a)

,

worin $R^1$, $R^2$, $R^3$, $R^4$ und X die oben angegebenen Bedeutungen haben, mit einem Reduktionsmittel in Gegenwart inerter Lösungsmittel zur Reaktion gebracht werden
oder
c) in dem Fall, in dem A

$$-\overset{|}{C}H-O-\overset{\overset{O}{\|}}{C}-R^5$$

ist,
Verbindungen der Formel (I-b)

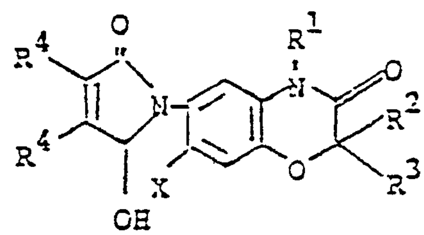

(I-b)

,

worin $R^1$, $R^2$, $R^3$, $R^4$ und X die oben angegebenen Bedeutungen haben, mit Verbindungen der Formel (IV)

$$R^5-\overset{\overset{O}{\|}}{C}-Hal \qquad (IV),$$

in der $R^5$ die oben angegebenen Bedeutungen hat und Hal ein Halogen-Atom bezeichnet, oder mit Verbindungen der Formel (V)

$$R^5-\overset{\overset{O}{\|}}{C}-O-\overset{\overset{O}{\|}}{C}-R^5 \qquad (V),$$

in der $R^5$ die oben angegebenen Bedeutungen hat, in Gegenwart inerter Lösungsmittel und gegebenenfalls in Gegenwart säurebindender Mittel zur Reaktion gebracht werden.

**6.** Herbizide Zusammensetzungen, dadurch gekennzeichnet, daß sie wenigstens ein Dialkylmaleinimid der Formel (I) gemäß den Ansprüchen 1 und 5 enthalten.

**7.** Verfahren zur Bekämpfung von Unkräutern, dadurch gekennzeichnet, daß Dialkylmaleinimide der Formel (I) gemäß den Ansprüchen 1 und 5 auf Unkräutern und/oder deren Lebensraum zur Einwirkung gebracht werden.

8. Verwendung von Dialkylmaleinimiden der Formel (I) gemäß den Ansprüchen 1 und 5 zur Bekämpfung von Unkräutern.

9. Verfahren zur Herstellung herbizider Zusammensetzungen, dadurch gekennzeichnet, daß Dialkylmaleinimide der Formel (I) gemäß den Ansprüchen 1 und 5 mit Streckmitteln und/oder grenzflächenaktiven Mitteln vermischt werden.

**Claims**

1. Dialkylmaleimides of the formula (I)

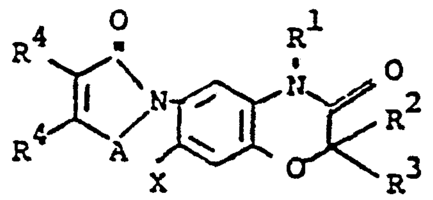

(I)

in which

A denotes

$$-\overset{\overset{O}{\|}}{C}-,\quad -\overset{\overset{OH}{|}}{C}H-\quad \text{or}\quad -\overset{|}{C}H-O-\overset{\overset{O}{\|}}{C}-R^5,$$

in which $R^5$ represents an alkyl group having 1 to 4 carbon atoms or a phenyl group,

$R^1$ represents a hydrogen atom, an alkyl group having 1 to 4 carbon atoms, an alkenyl group having 2 to 4 carbon atoms, an alkinyl group having 2 to 4 carbon atoms, a benzyl group, an alkoxyalkyl group having a total of 2 to 6 carbon atoms, a cyanomethyl group, a trimethylsilylmethyl group, an alkylthioalkyl group having a total of 2 to 6 carbon atoms, or the group

$$-\overset{\overset{R^6}{|}}{C}H-COR^7$$

in which $R^6$ represents a hydrogen atom or an alkyl group having 1 to 2 carbon atoms and $R^7$ represents an alkoxy group having 1 to 4 carbon atoms, a cycloalkoxy group having 3 to 7 carbon atoms, a halogenoalkoxy group having 1 to 4 carbon atoms, an alkylamino or dialkylamino group having a total of 2 to 6 carbon atoms, an N-($C_1$-$C_4$) alkyl-N-phenylamino group or a trimethylsilylmethoxy group,

$R^2$ denotes a hydrogen atom or an alkyl group having 1 to 6 carbon atoms, a phenyl group or a benzyl group,

$R^3$ denotes a hydrogen atom or an alkyl group having 1 to 4 carbon atoms,

$R^4$ denotes an alkyl group having 1 to 4 carbon atoms, and

X denotes a hydrogen atom, a fluorine atom, a chlorine atom, a bromine atom, a methyl group or an ethyl group.

2. Compounds according to Claim 1, characterised in that

A denotes

$$-\overset{\overset{O}{\|}}{C}-,\quad -\overset{\overset{OH}{|}}{C}H-\quad \text{or}\quad -\overset{|}{C}H-O-\overset{\overset{O}{\|}}{C}-R^5,$$

in which $R^5$ represents a methyl, ethyl or phenyl group,

$R^1$ denotes a hydrogen atom, a methyl, ethyl, propyl, allyl, propargyl, benzyl, methoxymethyl, cyanomethyl, trimethylsilylmethyl, methylthiomethyl or 2-ethylthioethyl group or the group

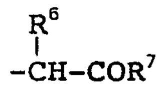

in which $R^6$ represents a hydrogen atom or a methyl group and $R^7$ represents a methoxy, ethoxy, cyclopentyloxy, cyclohexyloxy, trifluoroethoxy, isopropylamino, dimethylamino, N-methyl-N-phenylamino or a trimethylsilylmethoxy group,

$R^2$ denotes a hydrogen atom or a methyl, ethyl, npropyl, n-butyl, n-hexyl, phenyl or benzyl group,

$R^3$ denotes a hydrogen atom or a methyl group,

$R^4$ denotes a methyl group and

X denotes a hydrogen or a fluorine atom.

**3.** Compounds according to Claim 1, denoted by one of the formulae

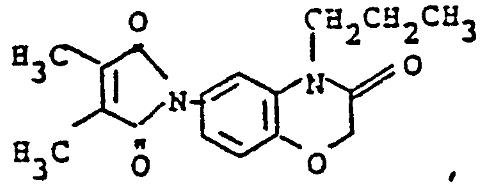

,

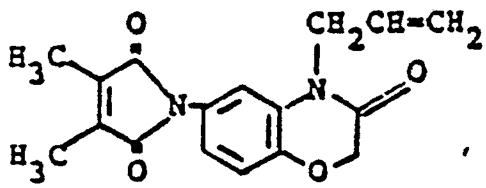

,

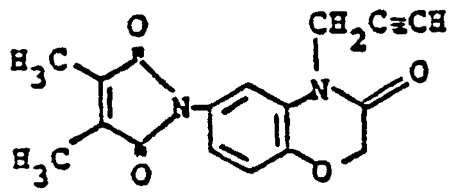

,

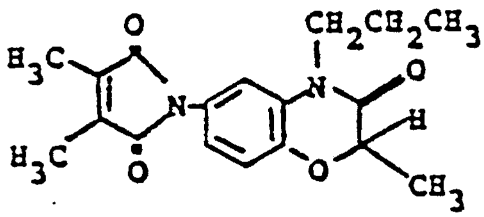

or

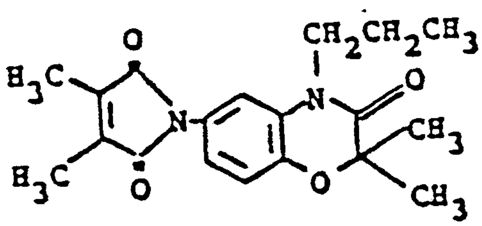

4. Dialkylmaleimides, denoted by one of the formulae

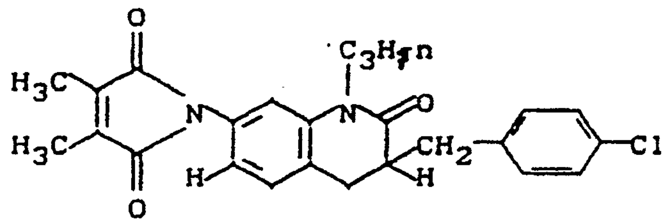

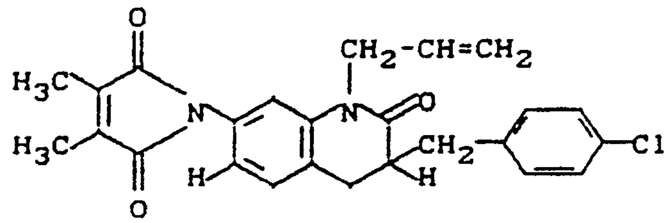

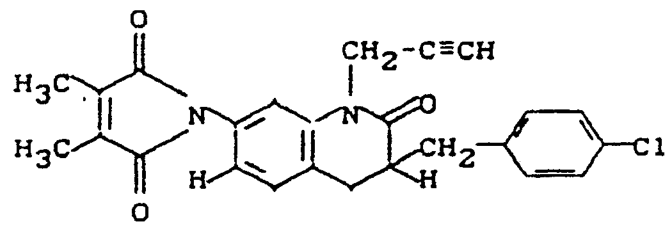

5. Process for the preparation of dialkylmaleimides of the formula (I)

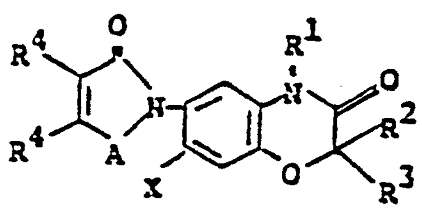

(I)

in which

A denotes

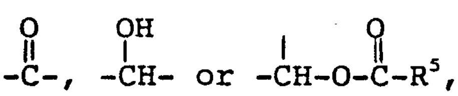

in which $R^5$ represents an alkyl group having 1 to 4 carbon atoms or a phenyl group,

$R^1$ represents a hydrogen atom, an alkyl group having 1 to 4 carbon atoms, an alkenyl group having 2 to 4 carbon atoms, an alkinyl group having 2 to 4 carbon atoms, a benzyl group, an alkoxyalkyl group having a total of 2 to 6 carbon atoms, a cyanomethyl group, a trimethylsilylmethyl group, an alkylthioalkyl group having a total of 2 to 6 carbon atoms, or the group

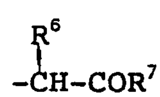

in which $R^6$ represents a hydrogen atom or an alkyl group having 1 to 2 carbon atoms and $R^7$ represents an alkoxy group having 1 to 4 carbon atoms, a cycloalkoxy group having 3 to 7 carbon atoms, a halogenoalkoxy group having 1 to 4 carbon atoms, an alkylamino or dialkylamino group having a total of 2 to 6 carbon atoms, an N-($C_1$-$C_4$) alkyl-N-phenylamino

group or a trimethylsilylmethoxy group,

$R^2$ denotes a hydrogen atom or an alkyl group having 1 to 6 carbon atoms, a phenyl group or a benzyl group,

$R^3$ denotes a hydrogen atom or an alkyl group having 1 to 4 carbon atoms,

$R^4$ denotes an alkyl group having 1 to 4 carbon atoms, and

X denotes a hydrogen atom, a fluorine atom, a chlorine atom, a bromine atom, a methyl group or an ethyl group,

characterised in that

a) in the case where A is

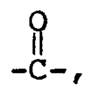

compounds of the formula (II)

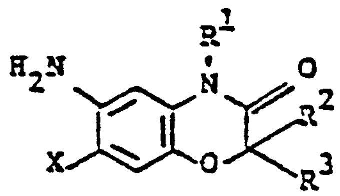

(II)

in which $R^1$, $R^2$, $R^3$ and X have the abovementioned meanings, are reacted with compounds of the formula (III)

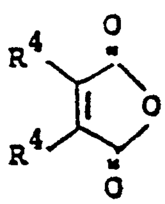

(III)

in which $R^4$ has the abovementioned meaning,
in the presence of inert solvents, or

b) in the case where A is

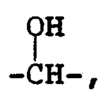

compounds of the formula (I-a)

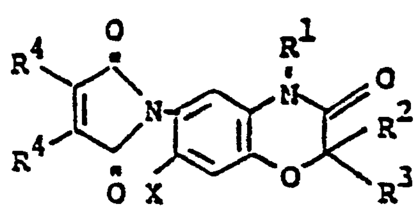

(I-a)

in which $R^1$, $R^2$, $R^3$, $R^4$ and X have the abovementioned meanings, are reacted with a reducing agent in the presence of inert solvents, or

c) in the case where A is

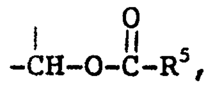

compounds of the formula (I-b)

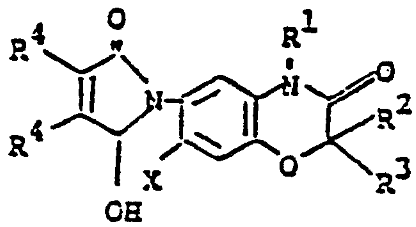

(I-b)

in which $R^1$, $R^2$, $R^3$, $R^4$ and X have the abovementioned meanings, are reacted with compounds of the formula (IV)

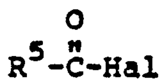

(IV)

in which $R^5$ has the abovementioned meanings and Hal denotes a halogen atom, or with compounds of the formula (V)

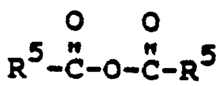

(V)

in which $R^5$ has the abovementioned meanings, in the presence of inert solvents and if appropriate in the presence of acid-binding agents.

**6.** Herbicidal compositions, characterised in that they contain at least one dialkylmaleimide of the formula (I) according to Claims 1 and 5.

**7.** Method of controlling weeds, characterised in that dialkylmaleimides of the formula (I) according to Claims 1 and 5 are allowed to act on weeds and/or their environment.

**8.** Use of dialkylmaleimides of the formula (I) according to Claims 1 and 5 for controlling weeds.

**9.** Process for the preparation of herbicidal compositions, characterised in that dialkylmaleimides of the formula (I) according to Claims 1 and 5 are mixed with extenders and/or surface-active agents.

**Revendications**

**1.** Dialkylmaléimides de formule I

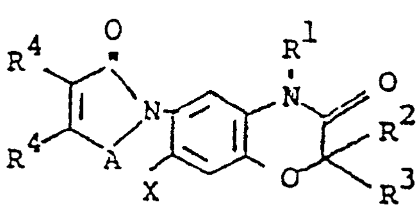

(I)

dans laquelle
A représente

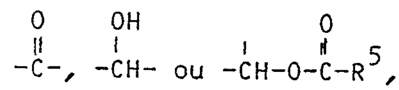
ou

dans lequel $R^5$ représente un groupe alkyle en $C_1$-$C_4$ ou un groupe phényle,
$R^1$ représente un atome d'hydrogène, un groupe alkyle en $C_1$-$C_4$, un groupe alcényle en $C_2$-$C_4$, un groupe alcynyle en $C_2$-$C_4$, un groupe benzyle, un groupe alcoxyalkyle contenant au total 2 à 6 atomes de carbone, un groupe cyanométhyle, un groupe triméthylsilylméthyle, un groupe alkylthioalkyle contenant au total 2 à 6 atomes de carbone ou
le groupe

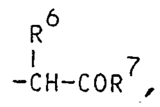

dans lequel $R^6$ représente un atome d'hydrogène ou un groupe alkyle en $C_1$-$C_2$ et $R^7$ un groupe alcoxy en $C_1$-$C_4$, un groupe cycloalcoxy en $C_3$-$C_7$, un groupe halogénoalcoxy en $C_1$-$C_4$, un groupe alkylamino ou dialkylamino contenant au total 2 à 6 atomes de carbone, un groupe N-(alkyle en $C_1$-$C_4$)-N-phénylamino ou un groupe triméthylsilylméthoxy,
$R^2$ représente un atome d'hydrogène ou un groupe alkyle en $C_1$-$C_6$, un groupe phényle ou un groupe benzyle,
$R^3$ représente un atome d'hydrogène ou un groupe alkyle en $C_1$-$C_4$,
$R^4$ représente un groupe alkyle en $C_1$-$C_4$, et
X représente un atome d'hydrogène, de fluor, de chlore, de brome, un groupe méthyle ou un groupe éthyle.

**2.** Composés selon la revendication 1, caractérisés en ce que :

A représente

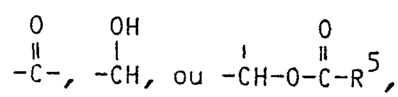
ou

dans lequel $R^5$ représente un groupe méthyle, éthyle ou phényle,
$R^1$ représente un atome d'hydrogène, un groupe méthyle, éthyle, propyle, allyle, propargyle, benzyle, méthoxyméthyle, cyanométhyle, triméthylsilylméthyle, méthylthiométhyle ou 2-éthylthioéthyle, ou
le groupe

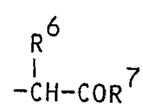

dans lequel $R^6$ représente un atome d'hydrogène ou un groupe méthyle et $R^7$ représente un groupe méthoxy, éthoxy, cyclopentyloxy, cyclohexyloxy, trifluoréthoxy, isopropylamino, diméthylamino, N-méthyl-N-phénylamino ou un groupe triméthylsilylméthoxy,
$R^2$ représente un atome d'hydrogène ou un groupe méthyle, éthyle, n-propyle, n-butyle, n-hexyle, phényle ou benzyle,
$R^3$ représente un atome d'hydrogène ou un groupe méthyle,
$R^4$ représente un groupe méthyle, et
X représente un atome d'hydrogène ou de fluor.

**3.** Composés selon la revendication 1, répondant aux formules

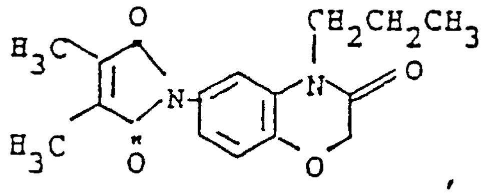

,

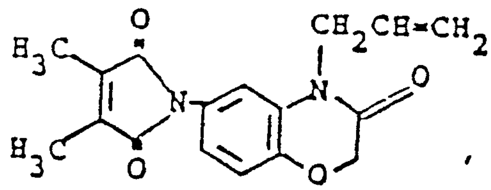

,

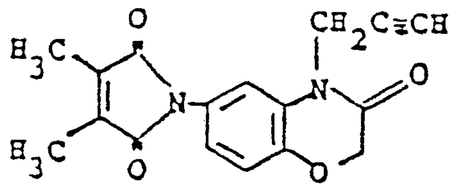

,

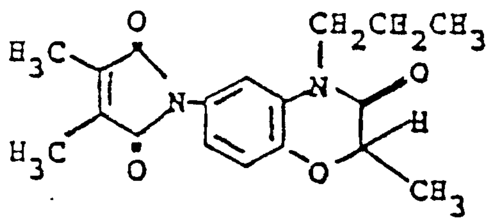

ou

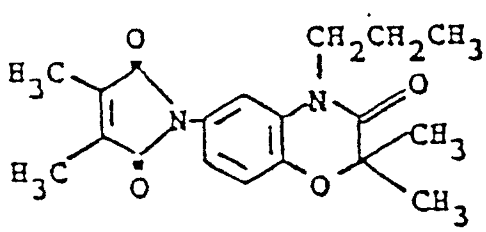

**4.** Dialkylmaléimides répondant à l'une des formules

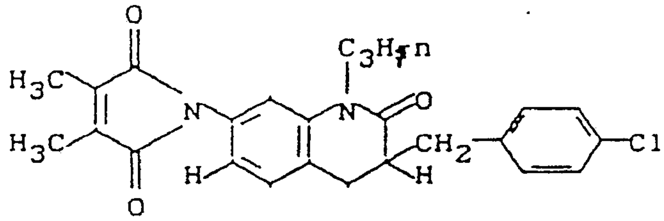

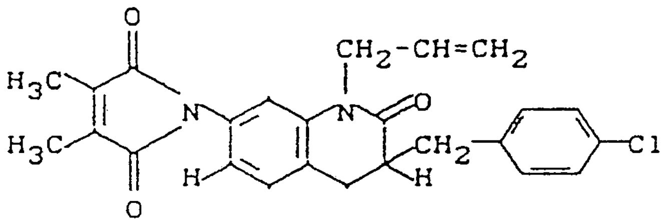

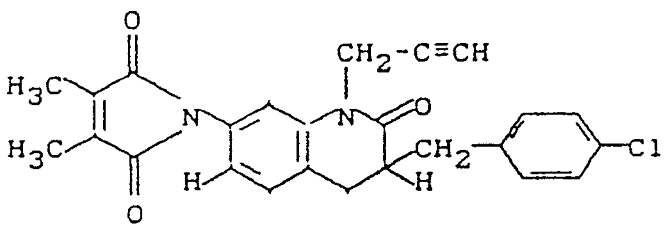

**5.** Procédé de préparation des dialkylmaléimides de formule I

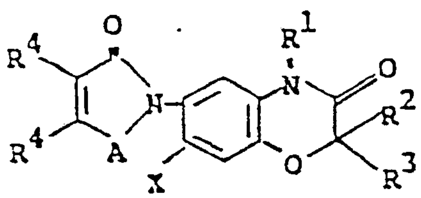

(I)

dans laquelle
A représente

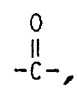, 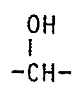 ou 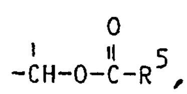,

dans lequel $R^5$ représente un groupe alkyle en $C_1$-$C_4$ ou un groupe phényle,
$R^1$ représente un atome d'hydrogène, un groupe alkyle en $C_1$-$C_4$, un groupe alcényle en $C_2$-$C_4$, un groupe alcynyle en $C_2$-$C_4$, un groupe benzyle, un groupe alcoxyalkyle contenant au total 2 à 6 atomes de carbone, un groupe cyanométhyle, un groupe triméthylsilylméthyle, un groupe alkylthioalkyle contenant au total 2 à 6 atomes de carbone ou
le groupe

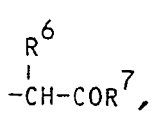,

dans lequel $R^6$ représente un atome d'hydrogène ou un groupe alkyle en $C_1$-$C_2$ et $R^7$ représente un groupe alcoxy en $C_1$-$C_4$, un groupe cycloalcoxy en $C_3$-$C_7$, un groupe halogénoalcoxy en $C_1$-$C_4$, un

groupe alkylamino ou dialkylamino contenant au total 2 à 6 atomes de carbone, un groupe N-(alkyle en $C_1$-$C_4$)-N-phénylamino ou un groupe triméthylsilylméthoxy,
$R^2$ représente un atome d'hydrogène ou un groupe alkyle en $C_1$-$C_6$, un groupe phényle ou benzyle,
$R^3$ représente un atome d'hydrogène ou un groupe alkyle en $C_1$-$C_4$,
$R^4$ représente un groupe alkyle en $C_1$-$C_4$, et
X représente un atome d'hydrogène, de fluor, de chlore, de brome, un groupe méthyle ou éthyle,
caractérisé en ce que :

a) au cas où A représente

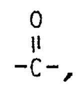

on fait réagir des composés de formule II

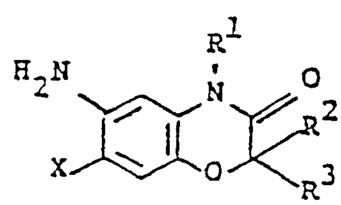

(II)

dans laquelle $R^1$, $R^2$, $R^3$ et X ont les significations indiquées ci-dessus, avec des composés de formule III

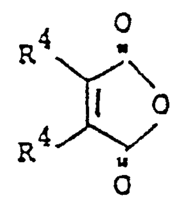

(III)

dans laquelle $R^4$ a les significations indiquées ci-dessus, en présence de solvants inertes, ou bien

b) au cas où A représente

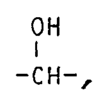

on fait réagir des composés de formule I-a

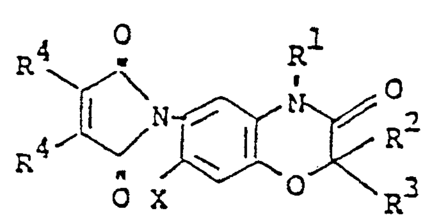

(I-a)

dans laquelle $R^1$, $R^2$, $R^3$, $R^4$ et X ont les significations indiquées ci-dessus, avec un agent réducteur en présence d'un solvant inerte, ou bien

c) au cas où A représente

$$-\overset{|}{C}H-O-\overset{O}{\overset{\|}{C}}-R^5,$$

on fait réagir des composés de formule I-b

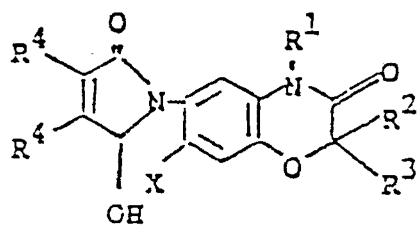

(I-b)

dans laquelle $R^1$, $R^2$, $R^3$, $R^4$ et X ont les significations indiquées ci-dessus, avec des composés de formule IV

$$R^5-\overset{O}{\overset{\|}{C}}-Hal \qquad (IV)$$

dans laquelle $R^5$ a les significations indiquées ci-dessus et Hal représente un atome d'halogène, ou avec des composés de formule V

$$R^5-\overset{O}{\overset{\|}{C}}-O-\overset{O}{\overset{\|}{C}}-R^5 \qquad (V)$$

dans laquelle $R^5$ a les significations indiquées ci-dessus, en présence de solvants inertes et le cas échéant en présence d'accepteurs d'acides.

6. Compositions herbicides, caractérisées en ce qu'elles contiennent au moins un dialkylmaléimide de formule I selon les revendications 1 et 5.

7. Procédé pour combattre les végétaux adventices, caractérisé en ce que l'on fait agir les dialkylmaléimides de formule I selon les revendications 1 et 5 sur les végétaux adventices et/ou leur habitat.

8. Utilisation des dialkylmaléimides de formule I des revendications 1 et 5 pour la lutte contre les végétaux adventices.

9. Procédé de préparation de compositions herbicides, caractérisé en ce que l'on mélange des dialkylmaléimides de formule I des revendications 1 et 5 avec des diluants et/ou des agents tensioactifs.